# EUROPÄISCHE PATENTSCHRIFT

(11) **EP 2 879 622 B1**
(45) Veröffentlichungstag und Bekanntmachung des Hinweises auf die Patenterteilung: **28.09.2016**
(21) Anmeldenummer: 13739228.8
(22) Anmeldetag: 19.07.2013
(51) Int. Cl.: A61F 2/60, A61F 2/66, A61F 2/76, A61F 2/50, A61F 2/70, A61F 2/72, B25J 9/00

(54) **ORTHESENSTEUERUNG**
CONTROL FOR ORTHOTIC DEVICES
COMMANDE D'ORTHÈSE

(30) Priorität: 02.08.2012 DE 102012107117
(43) Veröffentlichungstag der Anmeldung: 10.06.2015
(73) Patentinhaber: Otto Bock HealthCare GmbH, 37115 Duderstadt (DE)
(72) Erfinder: WÖRGÖTTER, Florentin, 37077 Göttingen (DE); BRAUN, Jan-Matthias, 37073 Göttingen (DE)
(74) Vertreter: Stornebel, Kai
(86) Internationale Anmeldenummer: PCT/EP2013/065285
(87) Internationale Veröffentlichungsnummer: WO 2014/019872

(56) Entgegenhaltungen:
- EP-A1- 1 442 703
- EP-A2- 0 628 296
- EP-B1- 1 058 524
- WO-A2-2006/024876
- DE-A1-102007 053 389

## Beschreibung

### TECHNISCHES GEBIET DER ERFINDUNG

Die Erfindung bezieht sich auf ein Verfahren zur Steuerung mindestens eines verstellbaren Aktuators einer Anschlusseinrichtungen an eine untere Gliedmaße aufweisenden orthopädischen Vorrichtung. Weiterhin bezieht sich die vorliegende Erfindung auf eine orthopädische Vorrichtung mit Anschlusseinrichtungen zum Anschluss an eine untere Gliedmaße und mit einer einen Aktuator ansteuernden Steuereinrichtung.

### DEFINITIONEN

Bei der orthopädischen Vorrichtung handelt es sich insbesondere um eine sogenannte Orthese, d. h. um eine Vorrichtung, die eine vorhandene Gliedmaße stützt. Die orthopädische Vorrichtung kann grundsätzlich aber auch eine sogenannte Prothese sein, die eine Gliedmaße zumindest teilweise ersetzt.

Unter einem Aktuator einer orthopädischen Vorrichtung ist hier jedes Mittel zu verstehen, das eine Relativbewegung zwischen Teilen der orthopädischen Vorrichtung beeinflusst. Diese Beeinflussung kann passiv, wie beispielsweise bei einem Dämpfer, oder auch aktiv sein, wie beispielsweise bei einem Motor.

Wenn im folgenden der Begriff "Bewegungsform" verwendet wird, so schließt er insbesondere die Bedeutung "Gangart" ein, ist hierauf aber auch bei einer unteren Gliedmaße nicht beschränkt.

### STAND DER TECHNIK

Bei einer gleichbleibenden Bewegungsform, beispielsweise dem Gehen in der Ebene oder dem Treppensteigen, die kontinuierlich abläuft, ist eine passende Einstellung des Aktuators einer orthopädischen Vorrichtung vergleichsweise einfach. Bei einem Wechsel der Bewegungsform ist jedoch auf eine andere Einstellung des Aktuators umzustellen. Diese Umstellung kann durch den Benutzer der orthopädischen Vorrichtung manuell vorgenommen oder mittels einer speziellen Bewegung der orthopädischen Vorrichtung ausgelöst werden. Eine für den Benutzer möglichst unbehinderte Bewegung mit einer orthopädischen Vorrichtung setzt aber voraus, dass die orthopädische Vorrichtung ihren Aktuator bzw. ihre Aktuatoren selbständig an die aktuelle Bewegungsform anpasst. Hierzu sind bereits verschiedenen Vorschläge unterbreitet worden.

Eine Beinprothese mit einem künstlichen Kniegelenk und ein Verfahren zur Steuerung einer Beinprothese sind aus der EP 1 058 524 B1 bekannt. Die Beinprothese weist einen Oberschenkelteil, einen Unterschenkelteil und ein die beiden verbindendes Kniegelenk auf. Das Kniegelenk umfasst ein Dämpfungselement zum Steuern der Kniegelenksbewegung. Eine Einrichtung erfasst den Kniewinkel und eine weitere Einrichtung die auf die Prothese wirkende Kraft. Eine Steuerung zum Steuern des Dämpfungselements in Abhängigkeit von den erfassten Werten für den Kniewinkel und für die Kraft wird entsprechend dem Gangverhalten des Trägers der Beinprothese geregelt. Dabei ist die Steuerung derart ausgebildet, dass sie das Dämpfungselement in Abhängigkeit von zuvor für verschiedene Ganggeschwindigkeiten gespeicherten Steuerparametern steuert. Diese Parameter werden im Rahmen der Regelung der Steuerung in Abhängigkeit von dem Gangverhalten nachgeregelt. Dazu wird als Funktion der erfassten Werte für den Kniewinkel und für die Kraft ein Steuerparameter aus den gespeicherten Steuerparametern ausgewählt. Der ausgewählte Steuerparameter wird dann als Funktion der erfassten Werte für den Kniewinkel und für die Kraft auf einen Wert geändert, der gleich groß ist wie oder anders als der vorher gespeicherte Wert. Dieser geänderte Steuerparameter wird dann anstelle des ausgewählten Steuerparameters gespeichert. Diese Regelung setzt ein, wenn ermittelte Ist-Schrittwerte von vorgegebenen Soll-Schrittwerten, die für jeden Prothesenträger gleich sind, abweichen. Dazu zählen beispielsweise ein maximaler Knickwinkel und eine Streckvoreilzeit, die die Zeit zwischen einem Extensionsanschlag und einem Aufsetzen der Ferse innerhalb eines Schrittes ist. Auf diese Weise wird die Steuerung des Dämpfungselements an den jeweiligen Prothesenträger angepasst.

Aus der EP 1 531 767 B1 sind eine Steuereinrichtung und ein Verfahren zum Steuern einer Prothese mit einem Aktuator bekannt. Dazu zählt ein Verfahren zum Bestimmen eines Fortbewegungsabschnitts und eines Fortbewegungsphasenabschnitts, um die Prothese in Echtzeit steuern zu können. Dieses Verfahren umfasst das Bereitstellen einer Mehrzahl von künstlichen Haupt-Propriozeptoren, das Empfangen eines Datensignals von jedem der künstlichen Haupt-Propriozeptoren, das Erhalten eines ersten und eines zweiten Ableitungssignals der Datensignale, das Erhalten eines dritten Ableitungssignals für zumindest manche der Datensignale und das Verwenden eines Satzes von ersten Zustandsmaschinen, um einen Zustand aus einer Mehrzahl möglicher Zustände für jeden künstlichen Haupt-Propriozeptor mit den entsprechenden Daten und Ableitungssignalen auszuwählen. Weiter wird der Fortbewegungsphasenabschnitt unter Verwendung der Zustände der künstlichen Haupt-Propriozeptoren erzeugt; und eine zweite Zustandsmaschine wird verwendet, um den Fortbewegungsabschnitt unter Verwendung von Ergebnissen, die den Datensignalen zugeordnet sind, aus einer Mehrzahl möglicher Fortbewegungsabschnitte auszuwählen. Das heißt, der Fortbewegungsabschnitt und der Fortbewegungsphasenabschnitt, d. h. Bewegungszustand und -phase, werden neben den aktuellen Werten der künstlichen Haupt-Propriozeptoren durch verschiedene Ableitungen dieser Werte erkannt und mit bestimmten Befehlen an die Aktuatoren der Prothese verknüpft. Hierzu werden die Signale der Haupt-Propriozeptoren bzw. deren Ableitungen mit Einträgen in Vergleichswertetabellen verglichen und, soweit eine Übereinstimmung vorliegt, die Aktuatoren durch mit diesen Einträgen in den Vergleichswertetabellen verknüpften Ansteuerbefehlen angesteuert. Die Einträge in den Vergleichswertetabellen sind dabei fest, d. h. nicht auf dem individuellen Prothesenträger abgestimmt.

Aus der DE 10 2007 053 389 A1, die den nächstliegenden Stand der Technik darstellt, ist ein Verfahren zur Steuerung eines orthopädischen Gelenks einer unteren Extremität in zumindest einem Freiheitsgrad mit einem verstellbaren Aktuator bekannt, das zur Anpassung einer orthopädischen Vorrichtung, die oberseitige Anschlussmittel an eine Gliedmaße und ein distal zu den Anschlussmitteln gelenkig angeordnetes orthopädisches Gelenk aufweist, an Gehsituationen dient, die von einem Gehen in der Ebene abweichen. Bei diesem bekannten Verfahren werden mehrere Parameter der orthopädischen Vorrichtung über Sensoren erfasst. Die erfassten Parameter werden mit Kriterien verglichen, die anhand mehrerer Parameter und/oder Parameterverläufe erstellt worden und in einer Rechnereinheit abgelegt sind. Ein Kriterium, das anhand der ermittelten Parameter und/oder Parameterverläufe geeignet ist, wird ausgewählt, und Bewegungswiderstände, Bewegungsumfänge, Antriebskräfte und/oder deren Verläufe werden in Abhängigkeit von dem gewählten Kriterium angepasst, um Sonderfunktionen zu steuern.

Aus der DE 10 2008 024 748 A1 sind eine Knieorthese sowie ein Verfahren zur Steuerung einer Knieorthese bekannt. Das Verfahren zur Steuerung der Knieorthese, die einer Oberschenkelstruktur, einer Gelenkeinrichtung und eine Unterschenkelstruktur mit einem Fußteil aufweist, sieht vor, das ein wirksames Moment, insbesondere ein Knöchelelement, innerhalb der Orthese bestimmt und der Widerstand der Aktuatoreinheit in Abhängigkeit von dem Moment verändert wird. Ergänzend kann die Aktuatoreinheit in Abhängigkeit von einem Kniemoment, dem Kniewinkel, oder der Raumorientierung zumindest einer Oberschenkel- oder Unterschenkelstruktur verändert werden. Dadurch ist es möglich, eine Kontrolle der Stand- und Schwungphase bei Patienten mit Lähmungen, bei denen das Bein nicht mehr willentlich kontrollierbar ist, zur Verfügung zu stellen. Durch das beispielsweise über einen hydraulischen Aktuator veränderliche Widerstandsmoment kann ein Kniemoment derart generiert werden, dass Standphasenflexion und alternierendes Bergab- und Treppabgehen ermöglicht werden. Mit aktiven Aktuatoreinheiten soll eine entsprechende Unterstützung der Bewegung erfolgen.

Aus der DE 10 2009 052 887 A1 ist ein Verfahren zur Steuerung eines orthetischen oder prothetischen Gelenks einer unteren Extremität bekannt. Das Gelenk weist eine Widerstandseinrichtung auf, der zumindest ein Aktuator zugeordnet ist, über den der Beuge- und/oder Streckwiderstand in Abhängigkeit von Sensordaten verändert wird. Über die Sensoren werden während der Benutzung des Gelenks Zustandsinformationen bereit gestellt. Die Sensordaten werden von zumindest einer Einrichtung zur Erfassung von zumindest zwei Momenten oder einem Moment und einer Kraft ermittelt. Die Sensordaten von mindestens zwei derart ermittelten Größen werden durch eine mathematische Operation miteinander verknüpft. Dadurch wird zumindest eine Hilfsvariable errechnet, die der Steuerung des Beuge- und/oder Streckwiderstands zugrunde gelegt wird.

Ein Verfahren mit den Merkmalen des Oberbegriffs des unabhängigen Patentanspruchs 1 und eine entsprechende orthopädische Vorrichtung mit den Merkmalen des Oberbegriffs des nebengeordneten Patentanspruchs 18 sind aus der DE 195 21 464 A1 bekannt. Hier geht es konkret um die Steuerung der Kniebremse eines Prothesen-Kniegelenks einer Oberschenkelprothese. Dabei wird ein computergesteuertes Bremsmoment in Abhängigkeit von der Gehbewegung des Prothesenträgers kontinuierlich zwischen "frei" und "blockiert" verändert, und die Gehbewegung wird durch gemessene EMG-Werte, im Fußbereich gemessene Druckwerte, durch den jeweiligen Kniewinkel sowie die jeweilige, zwischen Prothesenober- und -unterschenkel gemessene Winkelgeschwindigkeit in Form von Messdaten charakterisiert. Zur verbesserten Anpassung der Kniebremssteuerung an verschiedene natürliche Gangarten wird die jeweilige Gangart aus einer Anzahl vorgegebener, zuvor für den jeweiligen Prothesenträger ermittelten Gangarten durch Auswerten von zumindest einigen der Messdaten ermittelt. Dann wird ein dieser ermittelten Gangart zugeordnetes Steuerprogramm ausgewählt. Für jedes Steuerprogramm wird eine als Zeitspanne definierte Schrittperiode zwischen zwei aufeinanderfolgenden Fersen/Boden-Kontakten unterteilt in mehrere Phasen festgelegt, deren jeweiliger Endpunkt durch für diese Phase vorgegebene, jeweils übermittelte Messdaten bestimmt wird. Jeder Phase werden bestimmte, sich während dieser Phase ggf. ändernde Bremswerte zugeordnet, mit denen die Kniebremse beaufschlagt wird. Die Ermittlung der jeweils ausgeübten Gangart erfolgt durch Vergleich der übermittelten Messdaten mit für jede Gangart vorgegebenen Referenzdaten. Dabei soll es zweckmäßig sein, die Referenzdaten aus den für jede einzelne Gangart gemessenen EMG-Daten zu ermitteln. Das Basieren der Ermittlung der jeweils ausgeübten Gangart auf EMG-Daten setzt deren reproduzierbare Messung voraus. Bei manchen Patientengruppen ist aber die Aufnahme von EMG-Daten überhaupt nicht möglich. Zudem ist das Ermitteln der Gangarten durch Datenvergleich komplex und fehleranfällig. Praktisch haben sich das bekannte Verfahren und die entsprechende Oberschenkelprothese daher keine Verbreitung gefunden.

Aus S.D. Prentice et al.: Simple artificial neural network models can generate basic muscle activity patterns for human locomotion at different speeds, Exp. Brain Res (1998) 123:474-480, ist es bekannt, Muskelaktivierungsmuster beim Gehen mit Hilfe eines künstlichen neuronalen Netzwerks auf der Basis einer Sinusfunktion und einer Cosinusfunktion mit einer Periodenlängen von der Dauer eines Schrittes darzustellen.

Aus der DE 601 31 377 T2 ist ein geschwindigkeitsangepasstes und patientenangepasstes Steuerschema für eine Knieprothese mit den Merkmalen des Oberbegriffs des unabhängigen Patentanspruchs 1 bekannt. Das Steuerschema ist vorgesehen, um eine Stehphasendämpfung der von einem Patienten getragenen Knieprothese zu steuern. In einem Speicher der Knieprothese sind Korrelationen bzgl. sensorischer Daten und der Stehphasendämpfung gespeichert. Diese Korrelationen sind aufgrund von klinischen Untersuchungen von Amputierten unterschiedlicher Körpergröße festgelegt und kennzeichnen das Knieverhalten, wenn sich die Fußprothese mit dem Boden in Kontakt befindet. Sensorische Informationen werden in Verbindung mit diesen Korrelationen verwendet, um zu definieren, wie die Stehphasendämpfung beim Stehen, Gehen oder Laufen moduliert werden soll.

Aus der US 2009/0054996 A1 ist eine Knieprothese bekannt, die eine einstellbare Gelenkbewegungssteuereinheit zum automatischen Steuern des Gelenks aufweist. Eine elektronische Speichereinheit speichert eine Zielbeziehung zwischen einem kinetischen oder kinematischen Bewegungsparameter und der Gehgeschwindigkeit. Die Zielbeziehung definiert eine Anzahl von Werten des Bewegungsparameters, die jeweils mit einer unterschiedlichen Gehgeschwindigkeit verbunden sind. Ein Überwachungssystem erzeugt Überwachungssignale, die Gehgeschwindigkeitswerte und Werte des Bewegungsparameters wiedergeben, wie sie bei unterschiedlichen Gehgeschwindigkeiten auftreten. Ein Abstimmungssystem stimmt die Gelenkbewegungssteuereinheit automatisch ab, wenn die Überwachungssignale eine Abweichung von der Zielbeziehung anzeigen, um den Bewegungsparameter zurück in den Bereich eines Wertes zu bringen, der von der Zielbeziehung für die jeweilige Gehgeschwindigkeit definiert wird.

### AUFGABE DER ERFINDUNG

Es ist die Aufgabe der Erfindung, ein Verfahren zur Steuerung mindestens eines verstellbaren Aktuators einer orthopädischen Vorrichtung und eine orthopädische Vorrichtung aufzuzeigen, die die jeweilige Bewegungsform zuverlässig anhand einfach zu messender Bewegungsparameter erkennen.

### LÖSUNG

Die Aufgabe der Erfindung wird durch ein Verfahren zur Steuerung mindestens eines verstellbaren Aktuators einer orthopädischen Vorrichtung gelöst, das die Merkmale des unabhängigen Patentanspruchs 1 aufweist, sowie durch eine orthopädische Vorrichtung mit einem verstellbaren Aktuator und einer Steuereinrichtung, die die Merkmale des unabhängigen Patentanspruchs 15 aufweist. Die abhängigen Patentansprüche 2 bis 14 betreffen bevorzugte Ausführungsformen des erfindungsgemäßen Verfahrens.

### BESCHREIBUNG DER ERFINDUNG

Bei einem erfindungsgemäßen Verfahren zur Steuerung mindestens eines verstellbaren Aktuators einer Anschlusseinrichtungen an eine untere Gliedmaße aufweisenden orthopädischen Vorrichtung werden fortlaufend Istwerte von mindestens zwei Bewegungsparametern der orthopädischen Vorrichtung mit mindestens zwei Sensoren erfasst. Zwischen den Abfolgen der Istwerte der mindestens zwei Bewegungsparameter wird ein funktionaler Zusammenhang ermittelt. Dies heißt, dass der funktionale Zusammenhang zwischen der Abfolge der Istwerte des einen und der Abfolge der Istwerte des anderen der mindestens zwei Parameter ermittelt wird. Der funktionale Zusammenhang gibt so die Relativbeziehung dieser beiden und aller weiteren berücksichtigten Abfolgen von Istwerten von Bewegungsparametern an. Dieser funktionale Zusammenhang wird fortlaufend wiederholt mit funktionalen Zusammenhängen bei definierten Bewegungsformen verglichen, um jeweils die Bewegungsform auszuwählen, die am besten zu den erfassten Istwerten passt. Dann werden unter Verwendung einer für die am besten passende Bewegungsform definierten Abfolge von Sollwerten Steuersignale für den Aktuator generiert.

Bei nahezu allen Bewegungsformen lassen sich spezielle Zusammenhänge zwischen den verschiedenen Bewegungsparametern aufzeigen, die durch Sensoren an einer orthopädischen Vorrichtung erfasst werden können. Erfindungsgemäß werden diese Zusammenhänge als funktionale Zusammenhänge der Istwerte von mindestens und vorzugsweise zwei solchen Bewegungsparametern erfasst. Bei geeigneter Wahl der Bewegungsparameter sind diese funktionalen Zusammenhänge ihrer Istwerte für jede relevante Bewegungsform charakteristisch, d. h. durch Vergleich des aktuellen funktionalen Zusammenhangs mit den funktionalen Zusammenhängen definierter Bewegungsformen kann vergleichsweise einfach die am besten passende dieser Bewegungsformen als die aktuell gültige ausgewählt werden. Insbesondere ist dafür keinerlei zeitliche Skalierung, d. h. keine Anpassung an eine aktuelle Schrittgeschwindigkeit erforderlich. Wenn dann unter Verwendung einer für diese am besten passende Bewegungsform definierte Abfolge von Sollwerten Steuersignale für den Aktuator generiert werden, sind die resultierenden Einstellungen des Aktuators optimal an die aktuelle Bewegungsform der orthopädischen Vorrichtung bzw. ihres Trägers angepasst.

Im Schritt des Ermitteins des funktionalen Zusammenhangs kann konkret eine Funktion ermittelt werden, die die Abfolge der Istwerte des einen der mindestens zwei Bewegungsparameter auf die Abfolge der Istwerte des anderen der mindestens zwei Bewegungsparameter abbildet. Der zeitliche Verlauf dieser Funktion wird dann mit den zeitlichen Verläufen entsprechender Funktionen der definierten Bewegungsformen verglichen.

Wenn im Schritt des Ermitteins ihres funktionalen Zusammenhangs die Istwerte der mindestens zwei Bewegungsparameter als Kreisfunktion dargestellt werden, ist dies in Bezug auf den funktionalen Zusammenhang besonders anschaulich. Auch der Vergleich dieser Kreisfunktion mit denjenigen der definierten Bewegungsformen, um die am besten passende Bewegungsform auszuwählen, ist leicht nachvollziehbar und zudem mit bekannten Algorithmen, wie beispielsweise demjenigen des kleinsten quadratischen Fehlers, einfach durchzuführen. Die Darstellbarkeit als Kreisfunktion setzt, wenn keine vorherige Transformation der Istwerte zumindest eines der beiden Parameter erfolgen soll, zwei Bewegungsparameter voraus, deren Istwerte sich um etwa 90° phasenversetzt zwischen zwei Grenzwerten hin- und herbewegen. Solche Bewegungsparameter sind jedoch insbesondere mit Winkelsensoren an der orthopädischen Vorrichtung erfassbar. Konkret können die beiden Bewegungsparameter ein Hüftwinkel und ein Kniewinkel sein, die an der orthopädischen Vorrichtung gemessen werden.

Im Schritt des Generierens von Steuersignalen für den Aktuator wird die Abfolge der Sollwerte, die durch die jeweils als am besten passend ermittelte Bewegungsform vorgegeben wird, als Funktion der Abfolge der Istwerte mindestens eines der mindestens zwei Bewegungsparameter ausgegeben. Auf diese Weise wird die Abfolge der Sollwerte mit der Phase der tatsächlichen Bewegung synchronisiert. Wenn die Istwerte der beiden Bewegungsparameter als Kreisfunktion dargestellt wird, kann die Abfolge der Sollwerte insbesondere als Funktion des Winkels um den Ursprung der Kreisfunktion ausgegeben werden, unter dem die Istwerte beider Bewegungsparameter aktuell zu finden sind. Bei dieser Ausführungsform des erfindungsgemäßen Verfahrens ist bei der Verwendung der Sollwerte keine explizite zeitliche Skalierung oder Anpassung an aktuelle Schrittweiten erforderlich. Alle in dieser Richtung notwendigen Anpassungen erfolgen vielmehr implizit, indem beispielsweise ein schnellerer Fortschritt der Bewegungsphase eine Beschleunigung der Abfolge der Sollwerte zur Folge hat.

Alternativ oder zusätzlich kann die Abfolge mit Hilfe des Signals eines weiteren Sensors zeitlich synchronisiert, d. h. zu einem bestimmten Bewegungspunkt in Beziehung gesetzt werden. Diese Maßnahme kann zur phasenrichtigen Ausgabe der Steuersignale an den Aktuator ausreichend sein. Der weitere Sensor kann insbesondere ein Fuß- oder Fersendrucksensor sein, der die Bodenkontakte des Fußes des Benutzers oder der orthopädischen Vorrichtung selbst registriert.

Bei der Durchführung des erfindungsgemäßen Verfahrens müssen nicht immer ganze Bewegungszyklen und entsprechende Umläufe einer verwendeten Kreisfunktion abgewartet werden, um nach einer möglicherweise besser passenden neuen Bewegungsform zu suchen. Vielmehr kann, sobald eine Abweichung zwischen dem aktuellen funktionalen Zusammenhang der Ist-werte der Bewegungsparameter und dem funktionalen Zusammenhang bei der bisher als am besten passend ermittelten Bewegungsform darauf hinweist, dass der Träger der orthopädischen Einrichtung eine neue Bewegungsform eingeleitet hat, sofort das Auswählen einer neuen definierten Bewegungsform gestartet werden, die besser zu den aktuellen Abfolgen der Istwerte der Bewegungsparameter bzw. deren funktionalen Zusammenhang passt. Die daraus resultierende Auswahl einer neuen Bewegungsform ist fortlaufend zu aktualisieren, bis sie sich stabilisiert hat. Wenn der Aktuator bereits abhängig von einer derart frühzeitig ausgewählten Bewegungsform eingestellt wird, besteht die große Chance, dass er schon sehr früh optimal eingestellt wird. Hingegen ist die Gefahr, dass er wegen einer auf Basis einer zunächst kleinen Wertebasis falsch ausgewählten Bewegungsform völlig unpassend eingestellt wird, nur sehr gering.

In einer bevorzugten Ausführungsform des erfindungsgemäßen Verfahrens wird eine Abweichung der Istwerte des einen der mindestens zwei Bewegungsparameter von Erwartungswerten, die durch die Istwerte des anderen der mindestens zwei Bewegungsparameter und den zuletzt ermittelten funktionalen Zusammenhang zwischen den Abfolgen der Istwerte der mindestens zwei Bewegungsparameter vorgegeben werden, als Bewegungsfehler eingestuft, wenn diese Abweichung eine Dauer unterhalb einer vorgegebenen Grenzdauer aufweist und/oder die Werte während der Abweichung zu keiner der definierten Bewegungsformen innerhalb einer vorgegebenen Fehlergrenze passen. Dabei kann auch ein Intergral oder eine Summe der in festen Intervallen bestimmten Abweichungen über einen Zeitraum hinweg betrachtet werden.

Grundsätzlich kann auch ein Frequenzspektrum der Abweichung betrachtet werden, wobei dann, wenn die Abweichung eine dominante Frequenz oberhalb einer vorgegebenen Grenzfrequenz aufweist, auf einen Bewegungsfehler geschlossen wird. Mit der Frequenz der Abweichung ist hier insbesondere gemeint, dass sich die Abweichung der Istwerte des einen Bewegungsparameters von den entsprechenden Erwartungswerten oder die Istwerte selbst mit dieser Frequenz ändern.

Istwerte, die eine als Bewegungsfehler eingeordnete Abweichung aufweisen, werden nicht verwendet, um nach einer möglicherweise besser passenden Bewegungsform und einer zugehörigen Abfolge von Sollwerten für die Ansteuerung des Aktuators zu suchen. Vielmehr wird zumindest zunächst die bisher als am besten passend ausgewählte Bewegungsform beibehalten und überwacht, ob die Istwerte des mindestens einen Bewegungsparameter zu den Erwartungswerten zurückkehren, die durch die bisherige Bewegungsform und die Istwerte des anderen Bewegungsparameters gesetzt werden. Sobald dies der Fall ist, werden die Steuersignale für den Aktuator weiter unter Verwendung der Abfolge der Sollwerte zu der bisherigen Bewegungsform generiert.

In dieser Ausführungsform des erfindungsgemäßen Verfahrens wird typischerweise jedes Stolpern oder Straucheln, das die bisherige Abfolge der Istwerte der beiden Bewegungsparameter unterbricht, erkannt und ihm wird in praktikabler Weise in Bezug auf die Ansteuerung des Aktuators begegnet. Dazu können die Steuersignale für den Aktuator während des Vorliegens eines Bewegungsfehlers auf einen vorgegebenen Rückfallwert festgelegt werden. Ein solcher vorgegebener Rückfallwert ist nicht mehr von den Istwerten der beiden Bewegungsparameter abhängig. Er kann aber von der bislang am besten passenden Bewegungsform und/oder der bisherigen Abfolge der Istwerte abhängig sein. Beispielsweise kann beim Erkennen eines Stolperns die Dämpfung eines Gelenks der orthopädischen Vorrichtung gezielt auf einen vergleichsweise hohen Rückfallwert festgelegt werden.

Weiterhin ist es bei dem erfindungsgemäßen Verfahren bevorzugt, wenn die Bewegungsformen für den jeweiligen Träger der orthopädischen Vorrichtung individuell definiert werden. Dies bedeutet, dass die Bewegungsformen in Übereinstimmung mit den natürlichen Bewegungsformen des Trägers der orthopädischen Vorrichtung definiert werden. Damit soll insbesondere für Träger von Orthesen eine optimale Ansteuerung des Aktuators der Orthese auch bei Bewegungsformen sichergestellt werden, die keinem üblichen Schema entsprechen. Zu derartigen Bewegungsformen zählen beispielsweise solche, die sich durch Schonung eines verletzten Körperteils ergeben.

Die Definitionen der einzelnen Bewegungsformen können aufgrund von ihnen zugeordneten tatsächlichen funktionalen Zusammenhängen zwischen den Istwerten der Bewegungsparameter aktualisiert werden. Funktionale Zusammenhänge, zu denen eine zuvor definierte Bewegungsform am besten passt, sind die aktuellen Repräsentanten dieser Bewegungsform. Sie spiegeln insbesondere Veränderungen der Bewegungsform wider, wie sie sich beispielsweise bei rekonvaleszenten Trägern von Orthesen regelmäßig über den Rekonvaleszenzprozess hinweg einstellen. Daher ist es vorteilhaft, die mit der Zeit auftretenden Änderungen der einer Bewegungsform zugeordneten funktionalen Zusammenhänge in die Definition der Bewegungsform einfließen zu lassen.

Bei dem erfindungsgemäßen Verfahren werden mindestens zwei Bewegungsformen definiert. Dies können beispielsweise die Bewegungsformen sein, die dem Gehen in der Ebene und dem Treppensteigen zugeordnet sind. Vorzugsweise sind die Bewegungsformen aber auch noch darüber hinaus differenziert. Beispielsweise kann eine zusätzliche Bewegungsform dem schnellen Laufen oder dem Treppabsteigen zugeordnet werden. Einzelne Träger einer orthopädischen Vorrichtung können auch Interesse an einer Definition spezieller Bewegungsformen haben, um beispielsweise verschiedene Tänze tanzen zu können, wobei für jeden Tanz eine Bewegungsform definiert wird, die das erfindungsgemäße Verfahren über den zugehörigen funktionalen Zusammenhang der Bewegungsparameter erkennt und dann die für den jeweiligen Tanz optimalen Einstellungen des Aktuators der orthopädischen Vorrichtung vornimmt.

Ebenso wie die Bewegungsformen werden vorzugsweise auch die Abfolgen der Sollwerte für die definierten Bewegungsformen individuell für den jeweiligen Träger der orthopädischen Vorrichtung festgelegt, um ihm mit den aus den Sollwerten generierten Steuersignalen für den Aktuator eine maximale Unterstützung bei den von ihm gewollten Bewegungen zu geben. Diese individuelle Definition kann durch Fachpersonal mit Unterstützung durch Analysevorrichtungen erfolgen. Gewisse Veränderungen der Definition können aber auch von dem Träger der orthopädischen Vorrichtung selbst vorgenommen werden.

Zusätzlich zur Veränderung der Definition der Abfolgen der Sollwerte für die Einstellung des Aktuators, um eine grundsätzliche Optimierung oder Anpassung an langfristige Veränderungen der Bewegungsformen vorzunehmen, kann beim Generieren der Steuersignale für den Aktuator auf Basis der jeweiligen Abfolge der Sollwerte eine Historie der Istwerte mindestens eines der Bewegungsparameter und/oder eine Historie des funktionalen Zusammenhangs zwischen den Abfolgen der Istwerte der mindestens zwei Bewegungsparameter berücksichtigt werden. Beispielsweise können kleiner werdende Schrittweiten auf eine Ermüdung des Trägers der orthopädischen Vorrichtung oder zumindest eine zunehmende Anstrengung hinweisen, denen mit einer stärkeren Unterstützung durch die orthopädische Vorrichtung begegnet werden kann.

Hinweise beispielsweise auf eine Ermüdung oder Anstrengung des Trägers der orthopädischen Vorrichtung können aber auch dadurch erhalten werden, dass mindestens ein Zustandswert der Gliedmaße mit mindestens einem Zustandssensor erfasst wird und dass die für die am besten passende Bewegungsform definierte Abfolge von Sollwerten für die Einstellung des Aktuators unter Berücksichtigung der erfassten Zustandswerte angepasst wird.

Neben Zustandswerten der Gliedmaße können auch Umgebungszustandswerte, die den Zustand der Umgebung, in der die orthopädische Vorrichtung verwendet wird, beschreiben, beim Anpassen der für die am besten passende Bewegungsform definierten Abfolge von Sollwerten für die Einstellung des Aktuators berücksichtigt werden. Zu diesen Umgebungszustandswerten zählen beispielweise die Temperatur, ein Haftreibungskoeffizient des Untergrunds oder dergleichen.

Von besonderem Interesse für eine optimale Steuerung des Aktuators der orthopädischen Vorrichtung ist die Ableitung der Umgebungssteigung, das heißt die Veränderung der Steigung des Untergrunds auf dem die aktuelle Bewegung erfolgt. Soweit möglich, kann die Ableitung der Umgebungssteigung direkt gemessen werden, um Anpassungen der Steuersignale für den Aktuator vorzunehmen. Da sich diese Ableitung der Umgebungssteigung aber auch in den erfassten Istwerten der Bewegungsparametern der orthopädischen Vorrichtung niederschlägt, kann sie auch aus diesen ermittelt werden.

Bei einer erfindungsgemäßen orthopädischen Vorrichtung mit Anschlusseinrichtungen an eine untere Gliedmaße, mit einem verstellbaren Aktuator, mit mindestens zwei Sensoren, die Ist-werte von mindestens zwei Bewegungsparametern der orthopädischen Vorrichtung fortlaufend erfassen, und mit einer Steuereinrichtung, die die erfassten Werte analysiert und basierend darauf den Aktuator mit Steuersignalen einstellt, führt die Steuereinrichtung das erfindungsgemäße Verfahren aus. Alle bevorzugten Ausführungsformen des erfindungsgemäßen Verfahrens entsprechen daher bevorzugten Ausführungsformen der erfindungsgemäßen Vorrichtung. Vorteilhafte Weiterbildungen der Erfindung ergeben sich aus den Patentansprüchen, der Beschreibung und den Zeichnungen. Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile von erfindungsgemäßen Ausführungsformen zwingend erzielt werden müssen. Ohne dass hierdurch der Gegenstand der beigefügten Patentansprüche verändert wird, gilt hinsichtlich des Offenbarungsgehalts der ursprünglichen Anmeldungsunterlagen und des Patents Folgendes: weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Schritt oder Element die Rede ist, ist dies so zu verstehen, dass genau ein Schritt oder Element, zwei Schritte oder Elemente oder mehr Schritte oder Elemente vorhanden sind. Wenn hingegen die genaue Anzahl eines Merkmals angegeben werden soll, findet das Adjektiv "genau" vor dem jeweiligen Merkmal Verwendung. Diese Merkmale können durch andere Merkmale ergänzt werden oder die einzigen Merkmale sein, aus denen das jeweilige Verfahren oder die jeweilige Vorrichtung besteht.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt eine erfindungsgemäße orthopädische Vorrichtung in Form einer Knie-Knöchel-Fuß-Orthese.
- **Fig. 2**: ist ein Flussdiagramm zum Setup einer erfindungsgemäßen orthopädischen Vorrichtung.
- **Fig. 3**: ist ein Flussdiagramm zum Betrieb einer erfindungsgemäßen orthopädischen Vorrichtung nach dem erfindungsgemäßen Verfahren.
- **Fig. 4**: ist eine Auftragung verschiedener Bewegungsparameter (ganz oben), einer Gangphase (oben) und der erfindungsgemäß eingestellten relativen Steifigkeit eines Aktuators der Orthese gemäß Fig. 1 (unten) über der Zeit in "Time Steps" für eine gleichbleibende Bewegungsform; und
- **Fig. 5**: ist eine Auftragung von zwei Bewegungsparametern als Kreisfunktion.

### FIGURENBESCHREIBUNG

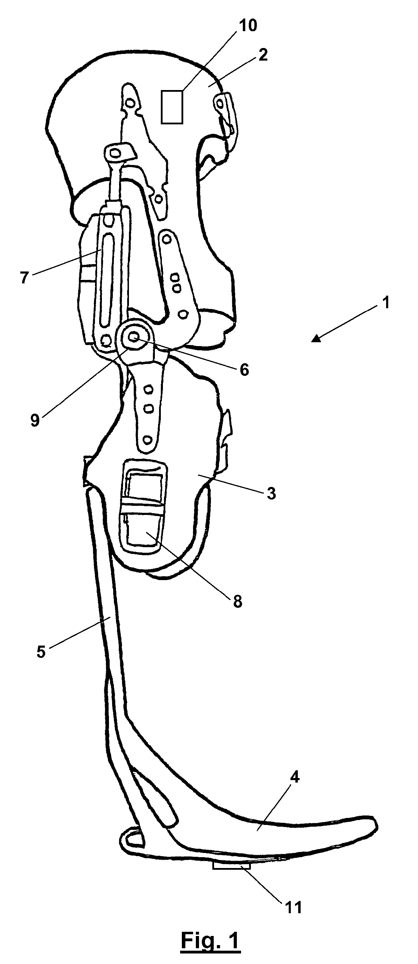

Die in **Fig. 1** in einer Seitenansicht schematisch dargestellte orthopädische Vorrichtung 1 ist eine Knie-Knöchel-Fuß-Orthese. Die orthopädische Vorrichtung 1 weist Anschlusseinrichtungen 2 bis 4 zum Anschluss an einen Oberschenkel, einen Unterschenkel und einen Fuß ihres Trägers auf. Zwischen der Anschlusseinrichtung 3 für den Unterschenkel und der Anschlusseinrichtung 4 für den Fuß ist ein flexibles Verbindungselement 5 angeordnet. Zwischen der Anschlusseinrichtung 2 für den Oberschenkel und der Anschlusseinrichtung 3 für den Unterschenkel ist ein Gelenk 6 vorgesehen. Dem Gelenk 6 ist ein einstellbarer Aktuator 7, beispielsweise in Form eines Dämpfers mit variabler Dämpfung zugeordnet, der die Relativbewegung der Anschlusseinrichtung 2 gegenüber der Anschlusseinrichtung 3 um das Gelenk 6 dämpft. Die Einstellung des Aktuators 7 wird durch Steuersignale von einer Steuereinrichtung 8 vorgenommen. Die Steuereinrichtung 8 berücksichtigt dabei Signale von Sensoren 9 bis 11, die in Fig. 1 nur schematisch angedeutet sind. Der Sensor 9 erfasst den Kniewinkel um das Gelenk 6. Der Sensor 10 erfasst den Hüftwinkel der Anschlusseinrichtung 2 gegenüber der Vertikalen; und der Sensor 11 erfasst den Bodenkontakt oder eine Bodenkontaktkraft. Es können auch noch weitere Sensoren an der orthopädischen Vorrichtung 1 vorgesehen sein, um weitere Bewegungsparameter an der orthopädischen Vorrichtung 1 oder auch Zustandsparameter der orthopädischen Vorrichtung 1, ihrer Umgebung oder ihres Trägers zu erfassen. Ebenso können weitere Aktuatoren, sowohl passive, wie der hier als Dämpfer ausgebildete Aktuator 7, als auch aktive, vorgesehen sein.

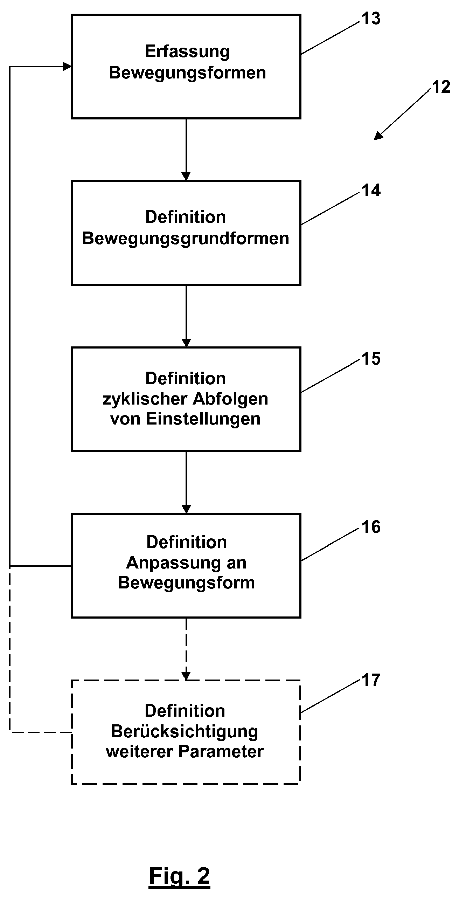

Um die Steuereinrichtung 8 der orthopädischen Vorrichtung 1 gemäß Fig. 1 für die Benutzung der orthopädischen Vorrichtung 1 durch einen hier nicht dargestellten Träger vorzubereiten, ist ein Setup 12 durchzuführen, das in **Fig. 2** skizziert ist. In einem ersten Schritt 13 werden die Bewegungen des Trägers der orthopädischen Vorrichtung beobachtet und Abfolgen von Istwerten der Bewegungsparameter registriert, die mit den Sensoren 9 bis 11 gemäß Fig. 1 erfasst werden. Dabei werden die Abfolgen der Istwerte bestimmten Bewegungsformen, wie beispielsweise dem Gehen in der Ebene oder dem Treppensteigen zugeordnet. Anhand von funktionalen Zusammenhängen der Abfolgen der Istwerte der verschiedenen Bewegungsparameter, werden die für jede dieser Bewegungsformen charakteristischen funktionalen Zusammenhänge ermittelt und damit in einem Schritt 14 die Bewegungsformen definiert, die jeweils mehreren sich beispielsweise nur in der Schrittgeschwindigkeit unterscheidenden Bewegungsformen entsprechen. Für diese verschiedenen Bewegungsformen werden dann in einem Schritt 15 optimale Einstellungen des Aktuators über jeden Zyklus der jeweiligen Bewegungsform hinweg definiert. Dies geschieht, indem jeweils eine Abfolge von Sollwerten für die Einstellungen des Aktuators festgelegt werden. Hieran schließt sich in einem Schritt 16 die Definition an, wie jeweils die Abfolge der Sollwerte zeitlich auf die Istwerte der Bewegungsparameter abgestimmt wird. Hierzu zählt beispielsweise eine Kopplung an die Abfolge der Istwerte eines der Bewegungsparameter oder eine punktuelle Synchronisation mit solchen Istwerten. In einem optionalen Schritt 17 können dann noch Berücksichtigungen weiterer Parameter beim Generieren der Steuersignale an den Aktuator auf Basis der Abfolgen der Sollwerte festgelegt werden. Hierzu zählt beispielsweise eine Anpassung der jeweiligen Abfolge der Sollwerte in Abhängigkeit von dem Signal eines zusätzlichen Muskelspannungssensors an der orthopädischen Einrichtung, der Umgebungstemperatur oder dergleichen.

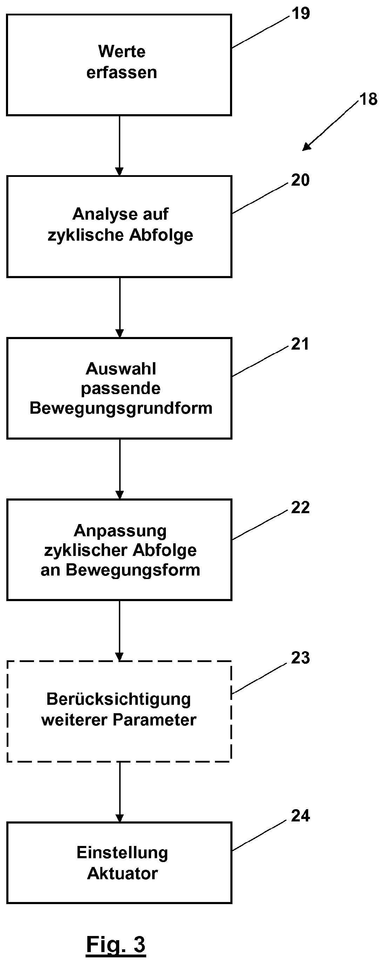

Der in **Fig. 3** skizzierte Betrieb 18 der orthopädischen Vorrichtung beginnt damit, dass in einem Schritt 19 die Istwerte der Bewegungsparameter mit den Sensoren 9 bis 11 gemäß Fig. 1 erfasst werden. In einem Schritt 20 werden die Abfolgen dieser Istwerte in der Steuereinrichtung 8 auf den zwischen ihnen vorliegenden funktionalen Zusammenhang analysiert. Anhand des ermittelten funktionalen Zusammenhang wird dann in einem Schritt 21 die zuvor in Schritt 14 gemäß Fig. 2 definierte Bewegungsform ausgewählt, deren funktionaler Zusammenhang die größten Übereinstimmungen mit dem aktuellen funktionalen Zusammenhang aufweist. In einem Schritt 22 werden auf Basis der Abfolge von Sollwerten, die der so ausgewählten definierten Bewegungsform im Schritt 15 gemäß Fig. 2 zugeordnet wurden Steuersignale für den Aktuator 7 gemäß Fig. 1 generiert. Dies erfolgt gemäß den Definitionen, die im Schritt 16 von Fig. 2 vorgenommen wurden. Entsprechend kann in einem Schritt 23 eine Berücksichtigung weiterer Parameter zur Anpassung der Abfolge der Sollwerte bzw. der daraus generierten Steuersignale gemäß den Definitionen des Schritts 17 in Fig. 2 erfolgen. Letztlich wird in einem Schritt 24 der Aktuator 7 gemäß Fig. 1 mit den Steuersignalen eingestellt. Die in Fig. 3 gezeigten Schritte werden fortlaufend wiederholt durchgeführt; und sie laufen in der Praxis nicht so ab, dass nur eine blockweise Verarbeitung ganzer Abfolgen von Istwerten oder Sollwerten erfolgt. Vielmehr werden die zu bestimmten Istwerten der Bewegungsparameter passenden Steuersignale so schnell als möglich an den 7 gemäß Fig. 1 ausgegeben. Dies bedeutet insbesondere, dass ein einmal auf Basis eines Sollwerts generiertes Steuersignal sofort ausgegeben wird. Entsprechend sind die Schritte 22 bis 24 praktisch nicht zu trennen.

Durch die Berücksichtigung der funktionalen Zusammenhänge der Bewegungsparameter und der daraus ermittelten passenden Bewegungsformen kann das erfindungsgemäße Verfahren dem Aktuator bereits für den jeweils nächsten Zeitpunkt geeignet einstellen, weil die potentielle Abweichung der aktuellen Istwerte der Bewegungsparameter von den aufgrund ausgewählten Bewegungsform zu erwartenden Werte, soweit kein Bewegungsfehler vorliegt, allenfalls minimal abweicht. Selbst bei einem Wechsel der Bewegungsform ist die physikalische Trägheit der unteren Gliedmaße, an die die orthopädische Vorrichtung angeschlossen ist, mindestens genauso hoch wie die Trägheit, mit der das erfindungsgemäße Verfahren eine Abweichung von der bisherigen Bewegungsform erkennt. Wenn durch auftretende Abweichungen der Übergang zu einer anderen Bewegungsform erkannt wird, sucht das erfindungsgemäße Verfahren sofort nach jetzt am besten passenden definierten Bewegungsform, wobei eine frühe Auswahl einer solchen Bewegungsform zunächst auch nur provisorisch erfolgen kann, bis sich die Auswahl als richtig oder falsch bestätigt. Im letzten Fall wird die zunächst ausgewählte Bewegungsform durch eine besser passende ersetzt.

Bei einem Bewegungsfehler, der allgemein dadurch definiert ist, dass der bisherige funktionale Zusammenhang zwischen den Istwerten der Bewegungsparameter nicht mehr besteht, aber zugleich Anzeichen dafür vorliegen, dass auch kein neuer funktionaler Zusammenhang vorliegt, der einer anderen definierten Bewegungsform entspricht, wird die Ansteuerung des Aktuators 7 gemäß Fig. 1 hingegen auf einen Rückfallwert festgelegt. Von dem Rückfallwert kehrt die Steuereinrichtung auf die Ansteuerung des Aktuators auf Basis der zu der bisher als am besten passend angesehenen Bewegungsform zugehörigen Abfolge von Sollwerten zurück, sobald die Istwerte der Bewegungsparameter wieder in den Bereich ihres bisherigen funktionalen Zusammenhangs zurückgekehrt sind. Auf diese Weise wird der Träger der orthopädischen Vorrichtung 1 gemäß Fig. 1 zum Beispiel in günstiger Weise unterstützt, wenn er ein Stolpern oder Straucheln zu bewältigen hat.

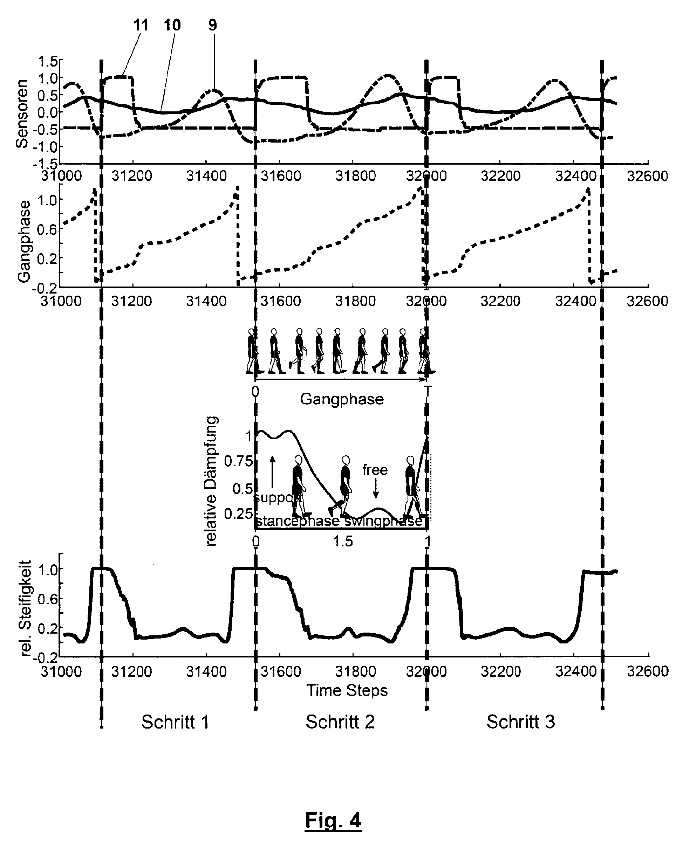

Für eine feste Bewegungsform zeigt **Fig. 4** ganz oben die zugehörigen Istwerte der mit den Sensoren 9 bis 11 gemäß Fig. 1 erfassten Bewegungsparameter. Die Istwerte des Kniewinkels, der mit dem Sensor 9 erfasst wird, sind mit einer Linie aus unterschiedlich langen Strichen wiedergegeben. Die Istwerte des Hüftwinkels, der mit dem Sensor 10 erfasst wird, sind mit durchgezogener Linie wiedergegeben; und der Bodenkontakt, der mit dem Sensor 11 erfasst wird, ist mit einer Linie aus gleich langen Strichen wiedergegeben. Die Istwerte zeigen zyklische Abfolgen, wobei jeder Zyklus einem Schritt entspricht. Innerhalb jedes Zyklus weisen sie Abfolgen der Istwerte des Hüftwinkels und des Kniewinkels einen gleichbleibenden funktionalen Zusammenhang auf, d. h. die Abfolgen können durch eine mathematische Funktion aufeinander abgebildet werden. Dieser funktionale Zusammenhang ist für die vorliegende Bewegungsform charakteristisch. Zudem kann aus dem Fortschritt dieser Funktion auf den Fortschritt der Gangphase geschlossen werden, der in Fig. 4 oben mit einer Linie aus kurzen Strichen unter den Werten der Sensoren dargestellt ist. Abhängig von dieser Gangphase wird dann die relative Dämpfung des Aktuators 7 gemäß Fig. 1 eingestellt. D. h. die zu der Bewegungsform zugehörige Abfolge von Sollwerten für die Einstellung des Aktuators wird phasenrichtig zur Gangphase zur Ausgabe von Steuersignalen an den Aktuator verwendet. Hierdurch wird die in Fig. 4 unten mit durchgezogener Linie dargestellte relative Steifigkeit des Aktuators 7 und damit des Gelenks 6 gemäß Fig. 1 eingestellt. Diese Einstellungen sind insbesondere auf die in Fig. 4 unten angegebenen Teilphasen der Gangphase, d. h. die Standsphase ("stancephase") und Schwingphase ("swingphase") abgestimmt. Während in der Standphase das Gelenk 6 gemäß Fig. 1 zur Unterstützung des Trägers steifer ist ("support"), lässt es in der Schwingphase ein freies Schwingen des Unterschenkels zu ("free").

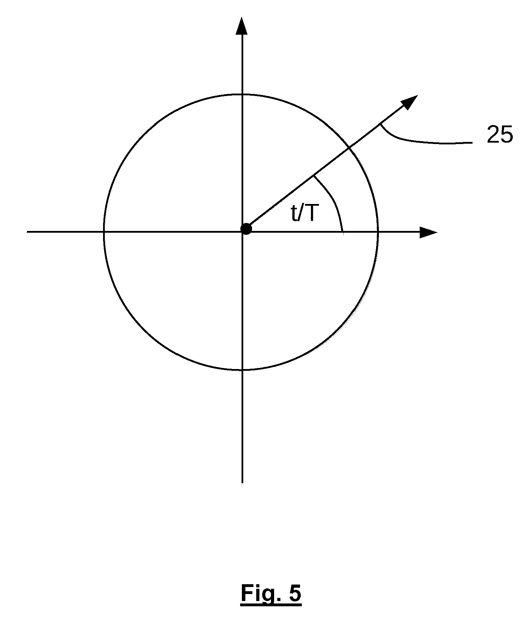

**Fig. 5** skizziert die Auftragung des Kniewinkels (horizontal) und des Hüftwinkels (vertikal) als Kreisfunktion über der Gangphase t/T, wobei T die Schrittdauer ist. Eine solche Auftragung als Kreisfunktion ist durch den Phasenversatz zwischen dem Kniewinkel und dem Hüftwinkel grundsätzlich auch direkt möglich. Hier sind der Kniewinkel und der Hüftwinkel jedoch nicht direkt aufgetragen sondern entsprechende Ausgaben eines künstlichen neuronalen Netzwerks, in das neben dem Kniewinkel und dem Hüftwinkel der Zeitpunkt der mit dem Sensor 11 erfassten Bodenkontakte eingegeben wird. Zumindest sind eine Skalierung des Kniewinkels und des Hüftwinkels sowie eine Phasenabstimmung sinnvoll. Die zeitgleichen Istwerte der beiden Winkel geben die Lage des Phasenzeigers 25 vor und zeigen damit unmittelbar den Fortschritt der Gangphase an. Dies ermöglicht es, die Abfolge der Sollwerte für die Einstellung des Aktuators an die in Fig. 5 dargestellte Kreisfunktion zu koppeln, um eine optimale Synchronisierung dieser Abfolge mit dem Fortschritt der Gangphase zu erreichen. Zudem zeigt die Darstellung als Kreisfunktion gemäß Fig. 5 direkt den funktionalen Zusammenhang zwischen dem Kniewinkel und dem Hüftwinkel auf. Auch ein Vergleich des aktuellen funktionalen Zusammenhangs zwischen diesen Bewegungsparametern mit den funktionalen Zusammenhängen definierter Bewegungsformen kann direkt über die entsprechenden Kreisfunktionen und deren Abweichungen voneinander erfolgen. Ebenso wird die Definition der verschiedenen Bewegungsformen durch die Visualisierung des funktionalen Zusammenhangs in der Kreisfunktion erleichtert.

### BEZUGSZEICHENLISTE

- 1: Orthopädische Vorrichtung
- 2: Anschlusseinrichtung
- 3: Anschlusseinrichtung
- 4: Anschlusseinrichtung
- 5: Verbindungselement
- 6: Gelenk
- 7: Aktuator
- 8: Steuereinrichtung
- 9: Sensor
- 10: Sensor
- 11: Sensor
- 12: Setup
- 13: Schritt
- 14: Schritt
- 15: Schritt
- 16: Schritt
- 17: Schritt
- 18: Betrieb
- 19: Schritt
- 20: Schritt
- 21: Schritt
- 22: Schritt
- 23: Schritt
- 24: Schritt
- 25: Phasenzeiger

## Patentansprüche

1. Verfahren zur Steuerung mindestens eines verstellbaren Aktuators (7) einer Anschlusseinrichtungen (2-4) an eine untere Gliedmaße aufweisenden orthopädischen Vorrichtung (1) mit den fortlaufend wiederholt ausgeführten Schritten:
- Erfassen von Istwerten von mindestens zwei Bewegungsparametern der orthopädischen Vorrichtung mit mindestens zwei Sensoren (9-11),
- Vergleichen der erfassten Istwerte mit definierten Bewegungsformen, um die Bewegungsform auszuwählen, die am besten zu den erfassten Istwerten passt, und
- Generieren von Steuersignalen für den Aktuator (7) unter Verwendung einer für die am besten passende Bewegungsform definierten Abfolge von Sollwerten,
**dadurch gekennzeichnet, dass** der Schritt des Vergleichens der erfassten Istwerte mit definierten Bewegungsformen die Schritte aufweist:
- Ermitteln eines funktionalen Zusammenhangs zwischen einer Abfolge der Istwerte des einen und einer Abfolge der Istwerte des anderen der mindestens zwei Bewegungsparameter und
- Vergleichen dieses funktionalen Zusammenhangs mit funktionalen Zusammenhängen bei den definierten Bewegungsformen, um die Bewegungsform auszuwählen, die am besten zu den erfassten Istwerten passt.

2. Verfahren nach Anspruch 1, **dadurch gekennzeichnet, dass** im Schritt des Ermitteins des funktionalen Zusammenhangs eine Funktion ermittelt wird, die die Abfolge der Istwerte des einen der mindestens zwei Bewegungsparameter auf die Abfolge der Istwerte des anderen der mindestens zwei Bewegungsparameter abbildet.

3. Verfahren nach Anspruch 1 oder 2, **dadurch gekennzeichnet, dass** im Schritt des Ermittelns des funktionalen Zusammenhangs die Istwerte der mindestens zwei Bewegungsparameter als Kreisfunktion dargestellt werden.

4. Verfahren nach einem der vorhergehenden Ansprüche, **dadurch gekennzeichnet, dass** die mindestens zwei Bewegungsparameter ein Hüftwinkel und eine Kniewinkel sind.

5. Verfahren nach einem der vorhergehenden Ansprüche, **dadurch gekennzeichnet, dass** im Schritt des Generierens von Steuersignalen für den Aktuator die Abfolge der Sollwerte als Funktion der Abfolge der Istwerte mindestens eines der mindestens zwei Bewegungsparameter ausgegeben wird.

6. Verfahren nach einem der vorhergehenden Ansprüche, **dadurch gekennzeichnet, dass** die Abfolge der Sollwerte anhand des Signals eines weiteren Sensors synchronisiert wird.

7. Verfahren nach einem der vorhergehenden Ansprüche, **dadurch gekennzeichnet, dass** eine Abweichung der Istwerte des einen der mindestens zwei Bewegungsparameter von Erwartungswerten, die durch die Istwerte des anderen der mindestens zwei Bewegungsparameter und den zuletzt ermittelten funktionalen Zusammenhang zwischen den Abfolgen der Istwerte der mindestens zwei Bewegungsparameter vorgegeben werden, als Bewegungsfehler eingestuft wird, wenn:
- die Abweichung eine Dauer unterhalb einer vorgegebenen Grenzdauer aufweist und/oder
- die Werte während der Abweichung zu keiner der definierten Bewegungsformen innerhalb einer vorgegebenen Fehlergrenze passen.

8. Verfahren nach Anspruch 7, **dadurch gekennzeichnet, dass** bei einer als Bewegungsfehler eingestuften Abweichung die Steuersignale für den Aktuator (7), sobald die Istwerte des einen der mindestens zwei Bewegungsparameter wieder den Erwartungswerten entsprechen, wieder unter Verwendung der für die zuletzt am besten passende Bewegungsform definierten Abfolge von Sollwerten generiert werden.

9. Verfahren nach Anspruch 7 oder 8, **dadurch gekennzeichnet, dass** während einer als Bewegungsfehler eingestuften Abweichung die Steuersignale für den Aktuator (7) auf einen vorgegebenen Rückfallwert festgelegt werden.

10. Verfahren nach einem der vorhergehenden Ansprüche, **dadurch gekennzeichnet, dass** die Bewegungsformen für einen Träger der orthopädischen Vorrichtung (1) individuell definiert werden.

11. Verfahren nach einem der vorhergehenden Ansprüche, **dadurch gekennzeichnet, dass** Definitionen der einzelnen Bewegungsformen aufgrund von ihnen zugeordneten funktionalen Zusammenhängen aktualisiert werden.

12. Verfahren nach einem der vorhergehenden Ansprüche, **dadurch gekennzeichnet, dass** die Abfolgen von Sollwerten für die einzelnen Bewegungsformen für einen Träger der orthopädischen Vorrichtung (1) individuell definiert werden.

13. Verfahren nach einem der vorhergehenden Ansprüche, **dadurch gekennzeichnet, dass** die für die am besten passende Bewegungsform definierte Abfolge von Sollwerten unter Berücksichtigung
- einer Historie der Istwerte mindestens eines der Bewegungsparameter und/oder
- einer Historie des funktionalen Zusammenhangs zwischen den Abfolgen der Istwerte der mindestens zwei Bewegungsparameter angepasst wird.

14. Verfahren nach einem der vorhergehenden Ansprüche, **dadurch gekennzeichnet, dass** mindestens ein Zustandswert der Gliedmaße und/oder mindestens ein Umgebungszustandswert mit mindestens einem Zustandssensor erfasst wird, und dass die für die am besten passende Bewegungsform definierte Abfolge von Sollwerten unter Berücksichtigung der erfassten Zustandswerte und Umgebungszustandswerte angepasst wird.

15. Orthopädische Vorrichtung (1) mit:
- Anschlusseinrichtungen (2-4) an eine untere Gliedmaße,
- einem verstellbaren Aktuator (7),
- mindestens zwei Sensoren (9-11), die Istwerte von mindestens zwei Bewegungsparametern der orthopädischen Vorrichtung (1) fortlaufend erfassen, und
- einer Steuereinrichtung (8), die die erfassten Werte analysiert und basierend darauf den Aktuator (7) mit Steuersignalen einstellt,
**dadurch gekennzeichnet, dass** die Steuereinrichtung (8) das Verfahren nach einem der vorhergehenden Ansprüche ausführt.

## Claims

1. A method for controlling at least one adjustable actuator (7) of an orthopedic device (1) including connection apparatuses (2-4) to a lower limb, comprising the following steps carried out in a continuously repeating manner:
- registering actual values of at least two movement parameters of the orthopedic device using at least two sensors (9-11),
- comparing the registered actual values with defined movement patterns in order to select the movement pattern which fits best to the registered actual values, and
- generating control signals for the actuator (7) using a sequence of intended values defined for the best-fitting movement pattern,
**characterized in that** the step of comparing the registered actual values with defined movement patterns includes the following steps:
- establishing a functional relationship between a sequence of the actual values of the one of the at least two movement parameters and a sequence of the actual values of the other one of said parameters and
- comparing this functional relationship with functional relationships in the defined movement patterns in order to select the movement pattern which fits best to the registered actual values.

2. The method as claimed in claim 1, **characterized in that** a function is established during the step of establishing the functional relationship, which function maps the sequence of the actual values of the one of the at least two movement parameters onto the sequence of the actual values of the other one of the at least two movement parameters.

3. The method as claimed in claim 1 or 2, **characterized in that** the actual values of the at least two movement parameters are represented by a trigonometric function in the step of establishing the functional relationship.

4. The method as claimed in one of the preceding claims, **characterized in that** the at least two movement parameters are a hip angle and a knee angle.

5. The method as claimed in one of the preceding claims, **characterized in that** the sequence of the intended values is output as a function of the sequence of the actual values of at least one of the at least two movement parameters in the step of generating control signals for the actuator.

6. The method as claimed in one of the preceding claims, **characterized in that** the sequence of the intended values is synchronized on the basis of the signal from a further sensor.

7. The method as claimed in one of the preceding claims, **characterized in that** a deviation of the actual values of the one of the at least two movement parameters from expected values, which are predetermined by the actual values of the other one of the at least two movement parameters and the most recently established functional relationship between the sequences of the actual values of the at least two movement parameters, is classified as a movement error if:
- the deviation has a duration below a predetermined duration limit and/or
- the values during the deviation do not fit to any one of the defined movement patterns within a predetermined error limit.

8. The method as claimed in claim 7, **characterized in that**, in the case of a deviation classified as a movement error, the control signals for the actuator (7) are once again generated using the sequence of intended values defined for the most recent best-fitting movement pattern as soon as the actual values of the one of the at least two movement parameters once again correspond to the expected values.

9. The method as claimed in claim 7 or 8, **characterized in that** the control signals for the actuator (7) are set to a predetermined fall-back value during a deviation classified as a movement error.

10. The method as claimed in one of the preceding claims, **characterized in that** the movement patterns are defined individually for a wearer of the orthopedic device (1).

11. The method as claimed in one of the preceding claims, **characterized in that** definitions of the individual movement patterns are updated on the basis of functional relationships assigned thereto.

12. The method as claimed in one of the preceding claims, **characterized in that** the sequence of intended values for the individual movement patterns are defined individually for a wearer of the orthopedic device (1).

13. The method as claimed in one of the preceding claims, **characterized in that** the sequence of intended values defined for the best-fitting movement pattern is adapted taking into account
- a history of the actual values of at least one of the movement parameters and/or
- a history of the functional relationship between the sequences of the actual values of the at least two movement parameters.

14. The method as claimed in one of the preceding claims, **characterized in that** at least one state value of the limb and/or at least one surrounding state value is registered by at least one state sensor and **in that** the sequence of intended values defined for the best-fitting movement pattern is adapted taking into account the registered state values and surrounding state values.

15. An orthopedic device (1) comprising:
- connection apparatuses (2-4) to a lower limb,
- an adjustable actuator (7),
- at least two sensors (9-11), which continuously register actual values of at least two movement parameters of the orthopedic device (1), and
- a control apparatus (8) which analyzes the registered values and sets the actuator (7) using control signals on the basis thereof,
**characterized in that** the control apparatus (8) carries out the method as claimed in one of the preceding claims.

## Revendications

1. Procédé de commande d'au moins un actionneur (7) réglable d'un dispositif de raccordement (2-4) à un dispositif orthopédique (1) présentant des membres inférieurs, comportant les étapes effectuées de manière répétitive et continue :
- détection de valeurs réelles d'au moins deux paramètres de mouvement du dispositif orthopédique avec au moins deux capteurs (9-11),
- comparaison des valeurs réelles détectées avec des formes de mouvement définies pour choisir la forme de mouvement qui est la mieux adaptée aux valeurs réelles détectées, et
- génération de signaux de commande pour l'actionneur (7) en utilisant une séquence définie de valeurs de consigne pour la forme de mouvement la mieux adaptée,
**caractérisé en ce que** l'étape de la comparaison des valeurs réelles détectées avec des formes de mouvement définies présente les étapes suivantes :
- détermination d'une relation fonctionnelle entre une séquence des valeurs réelles de l'un desdits au moins deux paramètres de mouvement et d'une séquence des valeurs réelles de l'autre paramètre de mouvement, et
- comparaison de cette relation fonctionnelle avec des rapports fonctionnels dans le cas des formes de mouvement définies pour choisir la forme de mouvement qui est la mieux adaptée aux valeurs réelles détectées.

2. Procédé selon la revendication 1, **caractérisé en ce que** dans l'étape de la détermination de la relation fonctionnelle, on détermine une fonction qui reproduit la séquence des valeurs réelles de l'un desdits au moins deux paramètres de mouvement sur la séquence des valeurs réelles de l'autre desdits au moins deux paramètres de mouvement.

3. Procédé selon la revendication 1 ou 2, **caractérisé en ce que** dans l'étape de la détermination de la relation fonctionnelle, les valeurs réelles desdits au moins deux paramètres de mouvement sont représentées sous forme de fonction circulaire.

4. Procédé selon l'une des revendications précédentes, **caractérisé en ce que** lesdits au moins deux paramètres de mouvement sont un angle de hanche et un angle de genou.

5. Procédé selon l'une des revendications précédentes, **caractérisé en ce que** dans l'étape de génération de signaux de commande pour l'actionneur, la séquence des valeurs de consigne est fournie en tant que fonction de la séquence des valeurs réelles d'au moins un desdits au moins deux paramètres de mouvement.

6. Procédé selon l'une des revendications précédentes, **caractérisé en ce que** la séquence des valeurs de consigne est synchronisée à l'aide du signal d'un autre capteur.

7. Procédé selon l'une des revendications précédentes, **caractérisé en ce que** l'on classe comme erreur de mouvement un écart des valeurs réelles de l'un desdits au moins deux paramètres de mouvement par rapport à des valeurs escomptées, qui sont prédéterminées par les valeurs réelles de l'autre desdits au moins deux paramètres de mouvement et par la liaison fonctionnelle déterminée en dernier entre les séquences des valeurs réelles desdits au moins deux paramètres de mouvement, lorsque :
- l'écart présente une durée inférieure à une durée limite prédéterminée, et/ou
- les valeurs pendant l'écart ne correspondent à aucune des formes de mouvement définies dans une limite d'erreur prédéterminée.

8. Procédé selon la revendication 7, **caractérisé en ce que** dans le cas d'un écart classé comme erreur de mouvement, les signaux de commande pour l'actionneur (7), dès que les valeurs réelles dudit au moins un desdits au moins deux paramètres de mouvement correspondent de nouveau aux valeurs escomptées, sont de nouveau engendrés en utilisant la séquence de valeurs de consigne définie pour la forme de mouvement la mieux adaptée en dernier.

9. Procédé selon la revendication 7 ou 8, **caractérisé en ce que** pendant un écart classé comme erreur de mouvement, les signaux de commande pour l'actionneur (7) sont fixés à une valeur par défaut prédéterminée.

10. Procédé selon l'une des revendications précédentes, **caractérisé en ce que** les formes de mouvement sont définies individuellement pour un support du dispositif orthopédique (1).

11. Procédé selon l'une des revendications précédentes, **caractérisé en ce que** des définitions des formes de mouvement individuelles sont actualisées sur la base de liaisons fonctionnelles qui leur sont associées.

12. Procédé selon l'une des revendications précédentes, **caractérisé en ce que** les séquences de valeurs de consigne pour les formes de mouvement individuelles sont définies individuellement pour un support du dispositif orthopédique (1).

13. Procédé selon l'une des revendications précédentes, **caractérisé en ce que** la séquence de valeurs de consigne définie pour la forme de mouvement la mieux adaptée, est adaptée en tenant compte
- d'un historique des valeurs réelles d'au moins un des paramètres de mouvement, et/ou
- d'un historique de la liaison fonctionnelle entre les séquences des valeurs réelles desdits au moins deux paramètres de mouvement.

14. Procédé selon l'une des revendications précédentes, **caractérisé en ce qu'**au moins une valeur d'état des membres et/ou au moins une valeur d'état d'environnement est détectée au moyen d'au moins un capteur d'état, et **en ce que** la séquence de valeurs de consigne définie pour la forme de mouvement la mieux adaptée est adaptée en tenant compte des valeurs d'état et des valeurs d'état d'environnement détectées.

15. Dispositif orthopédique (1) comportant :
- des dispositifs de raccordement (2-4) à un membre inférieur,
- un actionneur (7) réglable,
- au moins deux capteurs (9-11) qui détectent en continu des valeurs réelles d'au moins deux paramètres de mouvement du dispositif orthopédique (1), et
- un dispositif de commande (8) qui analyse les valeurs détectées et règle, sur la base de celles-ci, l'actionneur (7) au moyen de signaux de commande,
**caractérisé en ce que** le dispositif de commande (8) met en oeuvre le procédé selon l'une des revendications précédentes.
